# EUROPEAN PATENT SPECIFICATION

(11) **EP 3 215 851 B1**
(45) Date of publication and mention of the grant of the patent: **15.07.2020**
(21) Application number: 15828333.3
(22) Date of filing: 03.11.2015
(51) Int. Cl.: G01N 33/574

(54) **LUNG CANCER SUB-TYPING METHOD**
LUNGENKREBSSUBTYPISIERUNGSVERFAHREN
PROCÉDÉ DE SOUS-TYPAGE DU CANCER DU POUMON

(30) Priority: 04.11.2014 GB 201419634
(43) Date of publication of application: 13.09.2017
(73) Proprietor: Randox Laboratories Ltd., Crumlin, County Antrim BT29 4QY (GB)
(72) Inventor: LOWRY, Philip, Crumlin Antrim BT29 4QY (GB); MCCONNELL, Ivan, Crumlin Antrim BT29 4QY (GB); FITZGERALD, Peter, Crumlin Antrim BT29 4QY (GB); CHACKO, Alex, Crumlin Antrim BT29 4QY (GB)
(86) International application number: PCT/EP2015/075556
(87) International publication number: WO 2016/071321

(56) References cited:
- WO-A1-2011/006642
- WO-A1-2011/109440
- WO-A2-2009/067546
- WO-A2-2013/096845
- SCHNEIDER JOACHIM ET AL: "Fuzzy logic-based tumor-marker profiles improved sensitivity in the diagnosis of lung cancer", INTERNATIONAL JOURNAL OF CLINICAL ONCOLOGY, CHURCHILL LIVINGSTONE JAPAN, TOKYO, GB, vol. 7, no. 3, 1 June 2002 (2002-06-01), pages 145-151, XP002403876, ISSN: 1341-9625
- G. Paone ET AL: "Discriminant analysis on small cell lung cancer and non-small cell lung cancer by means of NSE and CYFRA-21.1", EUROPEAN RESPIRATORY JOURNAL., vol. 8, no. 7, 1 July 1995 (1995-07-01), pages 1136-1140, XP055500549, DK ISSN: 0903-1936, DOI: 10.1183/09031936.95.08071136
- G Paone1 ET AL: "Validation of an algorithm able to differentiate small cell lung cancer from non-small-cell lung cancer patients by means of a tumour marker panel: analysis of errors", British Joumal of Cancer Cancer Research Campaign, 1 January 1997 (1997-01-01), pages 448-450, XP055500551, Retrieved from the Internet: URL:https://www.ncbi.nlm.nih.gov/pmc/artic les/PMC2063371/pdf/brjcancer00180-0142.pdf [retrieved on 2018-08-17]
- O. Taguchi ET AL: "Prognostic significance of plasma D-dimer levels in patients with lung cancer", Thorax, vol. 52, no. 6, 1 June 1997 (1997-06-01), pages 563-565, XP055500552, GB ISSN: 0040-6376, DOI: 10.1136/thx.52.6.563
- LISENBEE N P ET AL: "Evaluation of C-Reactive Protein Levels in the Diagnosis of Acute Pulmonary Embolism", ANNALS OF EMERGENCY MEDICINE, vol. 62, no. 4, 31 October 2013 (2013-10-31), XP028718480, ISSN: 0196-0644, DOI: 10.1016/J.ANNEMERGMED.2013.07.349
- AZIZ G?M?S ET AL: "A novel biomarker in the diagnosis of parapneumonic effusion: neutrophil gelatinase-associated lipocalin", MULTIDISCIPLINARY RESPIRATORY MEDICINE, BIOMED CENTRAL LTD, LONDON, UK, vol. 9, no. 1, 15 September 2014 (2014-09-15), page 49, XP021198235, ISSN: 2049-6958, DOI: 10.1186/2049-6958-9-49
- Shankar Siva ET AL: "A Pattern of Early Radiation-Induced Inflammatory Cytokine Expression Is Associated with Lung Toxicity in Patients with Non-Small Cell Lung Cancer", PLoS ONE, vol. 9, no. 10, 7 October 2014 (2014-10-07), page e109560, XP055500555, DOI: 10.1371/journal.pone.0109560
- FRANK EMMERT-STREIB ET AL: "Collectives of diagnostic biomarkers identify high-risk subpopulations of hematuria patients: exploiting heterogeneity in large-scale biomarker data", BMC MEDICINE, BIOMED CENTRAL LTD., LONDON, GB, vol. 11, no. 1, 17 January 2013 (2013-01-17), page 12, XP021139778, ISSN: 1741-7015, DOI: 10.1186/1741-7015-11-12

## Description

### FIELD OF THE INVENTION

This invention relates to the use of specific assays for diagnosing of solid tumours in human subjects. The invention also relates to determination of tumour type or sub-type in persons diagnosed with, or suspected of having, cancer.

### BACKGROUND OF THE INVENTION

Cancer is a condition whereby normal cells in various organs can progressively acquire a series of defects that result in their uncontrolled proliferation, and often migration. Cancerous cells have been defined as having up to eight key hallmarks which are acquired during the progression from healthy tissue through benign growths and eventually to malignant tumours [1]. These are; sustained proliferative signalling, blocking of growth suppressor signalling, resistance to death signals, induction of angiogenesis, acquiring replicative immortality, activation of invasive properties, deregulation of metabolic control, and evasion of immune destruction.

As cells acquire more genetic mutations and other defects, their level of transformation increases, and they acquire more of the properties listed above. They first progress from hyper-proliferative lesions to benign (non-malignant) tumours which grow slowly and do not metastasise (i.e. spread to other tissues). As further defects accumulate, they may eventually develop into a cancerous tumour. Malignant tumours can be both locally invasive and metastatic, and are often lethal.

The most common cancer worldwide is currently lung cancer, with 1.2 million new cases and 1.1 million deaths annually [2]. The incidence is highest in western countries, but is increasing rapidly in developing countries in line with tobacco consumption [2, 3]. Lung cancers can be grouped into a number of pathological subtypes, with the two major subtypes being non-small cell lung carcinoma (NSCLC) and small cell lung carcinoma (SCLC).

SCLC accounts for between 14% and 25% of lung cancer diagnoses [3, 4, 5], and is believed to originate in neuroendocrine cells. SCLC is highly correlated with smoking [4] and has a dismal prognosis; 75% of patients are diagnosed with extensive disease which has a median survival of 8-13 months and a 5-year survival rate of <5% [3]. NSCLC accounts for most of the remainder of lung cancers and is therefore the most prevalent subtype, however NSCLC is usually further sub-divided into squamous cell carcinoma, adenocarcinoma, and large cell carcinoma. Further subtypes of these NSCLC designations are sometimes applied, based on cytological features or molecular/genetic characterisation of the tumour.

One of the main reasons for the poor survival rates for all types of lung cancer is that patients typically present in the clinic when the tumour is already at an advanced stage. For example, Stage 1 NSCLC, where the tumour is less than 5cm in diameter and localised to the site of origin, has a 5-year survival rate of approximately 50% with treatment. Stages 3-4 NSCLC, where the tumour has spread beyond the lung of origin, the 5-year survival rate is less than 10% [www.cancerresearch.uk.org, accessed 12 June 2014]. Extensive stage SCLC, where the tumour has spread beyond the site of origin, has a 5-year survival of <5%, as stated above, but limited stage SCLC, where the tumour is confined to the original lung, has a 5 year survival of 20-25% with optimum treatment [3].

SCLC is generally treated with platinum-based chemotherapy, which has a very high response rate although most patients do eventually relapse. NSCLC may initially be treated with radiotherapy, chemotherapy, targeted therapeutics, or a combination of these. Lung adenocarcinoma, in particular, has a number of novel drugs available which may be appropriate depending on further molecular or cytological subtyping. Early stage disease for both NSCLC and SCLC is treatable by surgery; a full cure is possible for the minority of cases which are suitable for surgery.

SCLC and NSCLC have significant differences in cellular origin, histology, behaviour, and therapeutic options. Early and accurate diagnosis of tumour subtype is critical to maximising patient survival from both main types of lung cancer. Patients with suspicious symptoms will typically be referred for thoracic CT scan or other radiological testing. If a lesion appears consistent with lung cancer, diagnosis and subtyping of lung cancer is verified by cytological examination of a tissue sample obtained by lung endoscopy.

However, there are a number of problems associated with this: There may be delays in referral and availability for CT scans and / or endoscopy, which can be significant in a rapidly progressing disease. In addition, many lung cancer patients are elderly, in poor general health, are poorly compliant or are fearful of invasive procedures. These factors can delay or prevent cytological diagnosis. Even if patients are compliant, there is a significant cost to healthcare systems and insurance providers for these procedures. Endoscopy is an invasive procedure with significant discomfort and associated morbidity for the patient - some elderly patients or those with degenerative lung conditions may be too high risk for endoscopy and never receive a firm diagnosis of their condition.

A number of secreted biomarkers have been identified as being increased in SCLC but not NSCLC and being of possible use in differential diagnosis [6]. These have included ProGRP and ENO2 / NSE. However, no biomarkers have been shown individually to have sufficient diagnostic power to be recommended as standard practice for sub-diagnosis between SCLC and NSCLC [3].

WO2011109440 discloses methods to theranose a lung cancer based on biomarker signatures and WO2011006642 discloses Flap endonuclease 1 protein as a marker of lung cancer, neither mention differentiation between SCLC and NSCLC. Taguchi *et al* (1997) [7] discloses the prognostic significance of plasma levels of D-dimer for prognosis of lung cancer patients. Lisenbee *et al* (2013) [8] discloses D-dimer along with CRP as discriminatory markers for diagnosis of pulmonary embolism. Gümüs et al (2014) [9] disclose measurements of NGAL and CRP for the diagnosis of diseases causing pleural fluid including lung cancer and Siva *et al* (2014) [10] discloses a panel of biomarkers including VEGF for monitoring the course of lung cancer. WO2013096845 discloses a list of 371 protein biomarkers associated with lung cancer which are proposed for use in methods of diagnosis, classification and monitoring of various lung conditions including SCLC and NSCLC. They provide 13 and 15 biomarker panels for use in distinguishing benign and malignant conditions. WO2009067546 provides a vast amount of between 2-12 biomarker panels for differentiating between small cell and other tumours. Schneider *et al* (2002) [11] aimed to increase the diagnostic efficacy for both SCLC and NSCLC of four tumour markers and C-reactive protein (CRP) by fuzzy logic modelling. There has been another previous attempt at describing a multi-biomarker algorithm for SCLC sub-diagnosis [12, 13]. This included the marker ENO2 / NSE, which is perhaps the best known serum marker of SCLC [14], along with three general cancer markers carcinoembryonic antigen (CEA), cytokeratin-19 fragment (CYFRA21-1), and tissue polypeptide antigen (TPA). This approach applied a weighting formula to the marker concentrations in serum from lung cancer subjects, and achieved an overall correct classification rate of 93.1%. In that study 6 of 52 SCLC cases were misclassified, giving a sensitivity of 88.5%. There is therefore a critical clinical need for improved and more efficient methods of providing accurate confirmation of lung cancer, and for determination of tumour subtype in lung cancer. Detailed here is a method for rapid differential diagnosis of small cell lung cancer from non-small cell lung cancer, using a panel of blood-based protein biomarkers. The method of the current invention has been demonstrated to give sensitivity values of 100%. This would increase the proportion of patients receiving accurate diagnosis of lung tumour type, allowing for earlier commencement of treatment at lower cost to the healthcare provider, and ultimately improving lung cancer survival rates.

### REFERENCES

[1] Hanahan D. and Weinberg R.A. 2011. Cell 144, 646-674
[2] Ferlay J., et al. 2010. Int. J. Cancer 127, 2893-2917
[3] Steiber P., et al. 2006. In NACB: Practice Guidelines and Recommendations for the Use of Tumor Markers in the Clinic - Lung Cancer (Section 3P). www.nacb.org/Impg/tumor LMPG draft PDF.stm
[4] Kenfield S.A., et al. 2008. Tob. Control 17, 198-204
[5] Travis W.D. 2011. Clin. Chest Med. 32, 669-692
[6] Steiber P. and Holdenreider S. 2009, Cancer Biomarkers 6, 221-224
[7] Taguchi O., et al 1997, Thorax; 52:563-565.
[8] Lisenbee N.P., et al 2013, Annals of Emergency Medicine S25;62, No. 4S.
[9] Gümüs A., et al 2014, Multidisciplinary Respiratory Medicine, 9:49.
[10] Siva S., et al 2014, PLOS ONE, October 2014, 9 (10), e109560.
[11] Schneider, J., et al2002, Int J Clin Oncol 7:145-151.
[12] Paone G., et al. 1997. British Journal of Cancer 75, 448-450.
[13] Paone G., et al. 1995. Eur. Respir. J. 8, 1136-1140.
[14] Carney D.N., et al. 1982. Lancet 13, 583-585.
[15] Zweig and Campbell 1993. Clin. Chem. 39, 561-577.

### SUMMARY OF THE INVENTION

The present invention is based on the observation that patients with small cell lung cancer (SCLC) may be distinguished from patients with non-small cell lung cancer (NSCLC) by means of determining the concentration of two or more biomarkers in an ex vivo sample from a patient and establishing the significance of these levels. An algorithm or decision tree can be applied to a panel of protein biomarkers measured in serologous samples from these patients

The protein markers measured include all, or combinations of the following; Vascular Endothelial Growth Factor (VEGF), D-dimer, Neuron specific enolase (NSE), C-reactive protein (CRP), and Neutrophil gelatinase-associated lipocalin (NGAL). Preferably, the biomarker assays are carried out simultaneously using a multiplexed biochip array or arrays. This biomarker panel allows for accurate diagnosis of the correct lung cancer subtype by the clinician, and for timely initiation of the appropriate treatment programme. The present invention also provides a means of monitoring tumour progression in cases of lung cancer where these markers are detectable, and facilitates clinical and pharmaceutical research studies into lung cancer.

In a first aspect, the present invention is a method for differential diagnosis of lung cancer in a subject, involving measurement of the concentrations of some or all of VEGF, NSE, CRP, D-dimer, and NGAL in a sample obtained from the subject, and establishing the significance of these levels. This can be achieved, for example, by applying a decision tree or regression analysis based on optimum cut-off values for these protein concentrations, which indicates whether that subject should be assigned to the SCLC or NSCLC group based on that algorithm.

In a second aspect, the present invention is a solid state device for use in the differential diagnosis of lung cancer comprising a substrate coated with antibodies that individually bind with high specificity to CRP, D-dimer, and NGAL.

In a third aspect, the present invention is a method for measuring quantities of proteins relevant to the study of lung cancer in the blood of a subject, and monitoring experimental or treatment outcomes by following these protein levels at appropriate time points during the study.

### DESCRIPTION OF THE FIGURES

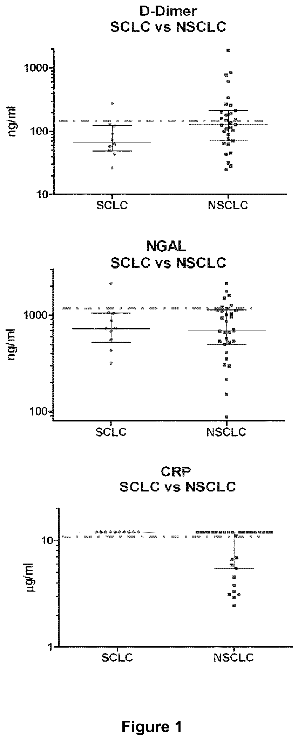

**Figure 1****:** The serum concentration of the markers shown was determined in a cohort of lung cancer patients. The markers shown were analysed simultaneously using a multiplexed biochip array and values plotted on a scatter plot. A total of 41 patient samples were analysed, of which 30 had been diagnosed with NSCLC and 11 with SCLC by gold standard cytology. Graphs are shown for **(A)** D-dimer, **(B)** NGAL, and **(C)** CRP. Bars show median and interquartile range for each analyte. Hatched lines show possible cut-off values applied to discriminate between conditions - the lines are by way of example and not necessarily indicative of actual cut-off applied.

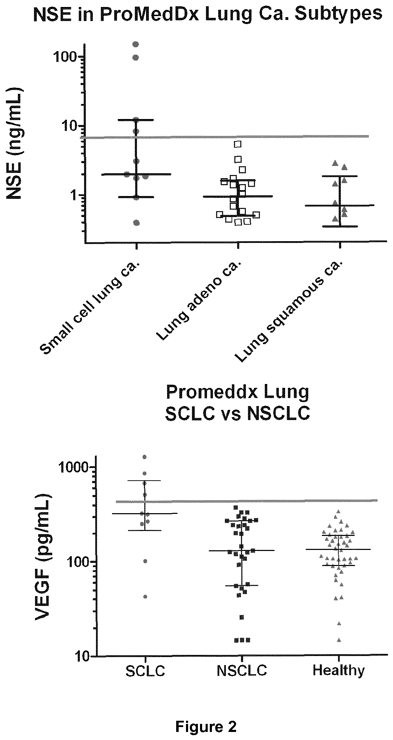

**Figure 2****:** The serum concentration of the markers shown was determined in a cohort of lung cancer patients. **(A)** VEGF was analysed by biochip independently of the other markers, while **(B)** NSE was analysed by multiplexed biochip simultaneously with analytes shown in Figure 1. For VEGF, values have been included for a group of healthy donors. Bars show median and interquartile range for each analyte. Hatched lines show possible cut-off values applied to discriminate between conditions - the lines are by way of example and not necessarily indicative of actual cut-off applied. For NSE, the NSCLC patients have been further sub-divided into adenocarcinoma and squamous cell carcinoma groups. These additional datasets shown do not affect the decision tree.

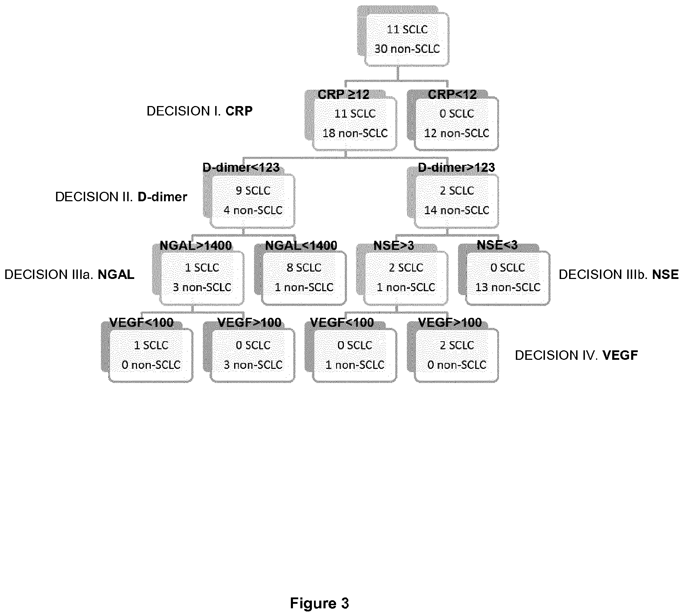

**Figure 3****:** Data was generated for the serum biomarkers mentioned above from a cohort of 41 lung cancer patient samples, of which 30 were NSCLC and 11 SCLC. Samples were stratified into the two groups by sequentially applying cut-off values for the markers in the sequence shown (see also the Detailed Description below). The cut-offs used in this example were 12 mg/L for CRP, 123 ng/ml for D-dimer, 1400 ng/ml for NGAL, 3 ng/ml for NSE and 100 pg/ml for VEGF. The algorithm was able to detect SCLC cases in this cohort with a sensitivity of 100% and a specificity of 96.7%, giving a positive predictive value of 91.7% and negative predictive value of 100%.

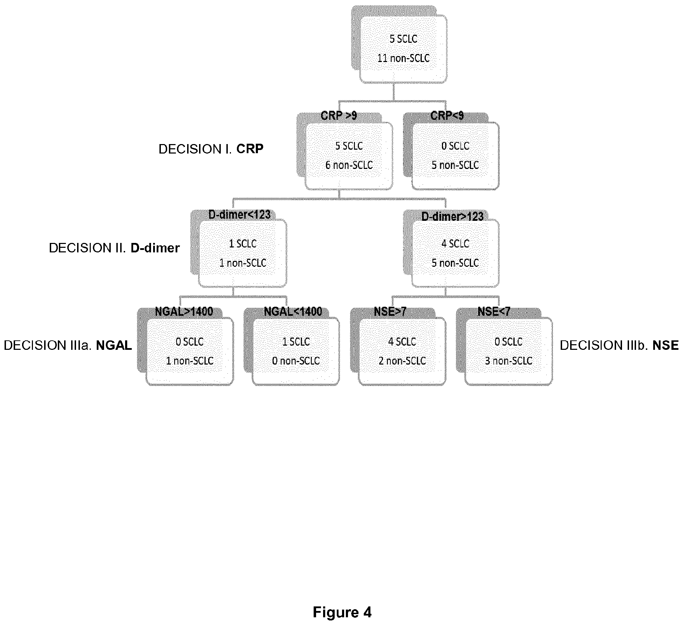

**Figure 4****:** Data was generated for the serum biomarkers mentioned above from an independent cohort of 16 lung cancer patient samples, of which 11 were NSCLC and 5 SCLC. Samples were stratified into the two groups by sequentially applying cut-off values for the markers in the sequence shown (see also the Detailed Description below). The cut-offs used in this example were 9 mg/L for CRP, 123 ng/ml for D-dimer, 1400 ng/ml for NGAL and 7 ng/ml for NSE. The algorithm was able to detect SCLC cases in this cohort with a sensitivity of 100% and a specificity of 81.8%, giving a positive predictive value of 71.4% and negative predictive value of 100%. The VEGF decision was not applied here since all SCLC cases had been classified by the first four markers.

### DETAILED DESCRIPTION OF THE INVENTION

Unless otherwise stated technical terms are used according to the conventional usage as known to those skilled in the art.

The present invention is based upon the observation that lung cancer can be subdivided into different subtypes. From the point of view of the patient, the symptoms and progression of different subtypes may be similar, and prognosis is generally poor no matter what the sub-diagnosis. However, subtypes of lung cancer have important cytological and molecular distinctions (reviewed by Travis, [5]), which allow for classification by the pathologist. Accurate and timely sub-classification of lung tumours are critical, since there are various treatment options including surgery, radiotherapy, chemotherapy, and novel targeted pharmaceuticals, which may be indicated depending upon the pathological classifications applied to the tumour.

The term "sensitivity", used in the context of a diagnostic test, describes the percentage or ratio of subjects actually positive for the condition that are deemed positive by the biomarker test, sometimes referred to as the true positive rate. The term "specificity", used in the context of a diagnostic test, indicates the percentage or ratio of the subjects deemed negative by the biomarker test that are actually negative for the condition (true negative rate). In these studies, it is customary for the amount of positive subjects to be pre-determined by the current gold standard of testing (in this case, cytology on biopsied tumour tissue), in order that these analyses may be performed.

Positive predictive value (PPV) and negative predictive value (NPV) are values that relate the sensitivity and specificity of the biomarker test to the actual prevalence of the disease states in the test population, and therefore give an impression of the likely utility of the biomarker test in practice. PPV is related to specificity, and describes the chance of being declared positive by gold standard if one receives a positive biomarker test.

NPV is related to sensitivity, and describes the chance of being negative by gold standard if one receives a negative biomarker test. If the prevalence of the test condition in the population is low (for instance having lung cancer in subjects recruited from the general population, a prevalence of ∼0.1% at any fixed time) [2], then the chance of a false positive is very high, and the PPV value is low. This means that many biomarker tests are not useful in practice unless test specificity is effectively 100%. The test condition described here, having SCLC from a population of subjects with lung cancer, has a prevalence of 15-25% [3, 4, 5].

Here, the inventors have described a panel of biomarkers for sub-diagnosis between SCLC and NSCLC. The inventors further describe an algorithm which, when applied to the biomarker data, is able to classify SCLC cases with very high sensitivity and specificity. The fact that a NPV of 100% was achieved for SCLC in both the test and verification sets is important clinically: This would allow for those cases in the negative category to be rapidly initiated on a treatment pathway for NSCLC, with certainty that this was appropriate. The PPV for SCLC (up to 81.8% here) may also be sufficiently high to allow treatment initiation for that disease with acceptable confidence.

The following definitions apply to terms used throughout this description and in relation to any of the aspects of the invention described herein.

In the context of the present invention, a "control" or "control value" is understood to mean the level of a biomarker typically found in patients who do not have cancer. The control level of a biomarker may be determined by analysis of a sample isolated from a person who does not have cancer or may be the level of the biomarker understood by the skilled person to be typical for such a person. The control value of a biomarker may be determined by methods known in the art and normal values for a biomarker may be referenced from the literature from the manufacturer of an assay used to determine the biomarker level. The "level" of a biomarker refers to the amount, expression level or concentration of the biomarker within the sample.

As used herein, references to "aiding diagnosis" may refer to aiding primary diagnosis, in the context of non-routine testing an individual to detect the presence of a disease, in particular cancer. In contrast to screening, in the context of diagnosis the patient is an individual who is presenting with symptoms of cancer or who has been motivated to seek medical attention due to symptoms of cancer. In preferred embodiments of the current invention, "aiding diagnosis" refers to stratification of a patient with a positive diagnosis of cancer, to further characterise the tumour.

As used herein, the term "a sample" includes biological samples obtained from a patient or subject, which may comprise blood, plasma, serum or urine. The methods of the invention described herein are carried out *ex vivo*. For the avoidance of doubt, the term "*ex vivo*" has its usual meaning in the art, referring to methods that are carried out in or on a sample obtained from a subject in an artificial environment outside the body of the subject from whom the sample has been obtained. The sample may be any sample taken from the subject from which the biomarkers of the current invention can be determined. Preferred samples include urine samples, blood samples, serum samples and plasma samples.

The terms "patient" and "subject" are used interchangeably herein and refer to any animal (e.g. mammal), including, but not limited to, humans, non-human primates, canines, felines, rodents and the like, which is to be the recipient of the diagnosis. Preferably, the subject or patient is a human.

The terms "immunoassay", "immuno-detection" and immunological assay" are used interchangeably herein and refer to antibody-based techniques for identifying the presence of or levels of a protein in a sample. Examples of such assays and methods are well known to those of skill in the art.

The term "antibody" refers to an immunoglobulin which specifically recognises an epitope on a target as determined by the binding characteristics of the immunoglobulin variable domains of the heavy and light chains (V_{H}S and V_{L}S), more specifically the complementarity-determining regions (CDRs). Many potential antibody forms are known in the art, which may include, but are not limited to, a plurality of intact monoclonal antibodies or polyclonal mixtures comprising intact monoclonal antibodies, antibody fragments (for example F_{ab}, F_{ab}', and Fᵣ fragments, linear antibodies, single chain antibodies and multi-specific antibodies comprising antibody fragments), single chain variable fragments (scF_{v'}s), multi-specific antibodies, chimeric antibodies, humanised antibodies and fusion proteins comprising the domains necessary for the recognition of a given epitope on a target. Preferably, references to antibodies in the context of the present invention refer to polyclonal or monoclonal antibodies. Antibodies may also be conjugated to various reporter moieties for a diagnostic effect, including but not limited to radionuclides, fluorophores, or dyes.

The term "binds specifically", in the context of antibody-epitope interactions, refers to an interaction wherein the antibody and epitope associate more frequently or rapidly, or with greater duration or affinity, or with any combination of the above, than when either antibody or epitope is substituted for an alternative substance, for example an unrelated protein. Generally, but not necessarily, reference to binding means specific recognition. Techniques known in the art for determining the specific binding of a target by a monoclonal antibody or lack thereof include but are not limited to, FACS analysis, immunocytochemical staining, immunohistochemistry, western blotting/dot blotting, ELISA, affinity chromatography. By way of example and not limitation, specific binding, or lack thereof, may be determined by comparative analysis with a control comprising the use of an antibody which is known in the art to specifically recognise said target and/or a control comprising the absence of, or minimal, specific recognition of said target (for example wherein the control comprises the use of a non-specific antibody). Said comparative analysis may be either qualitative or quantitative. It is understood, however, that an antibody or binding moiety which demonstrates specific recognition of a given target is said to have higher specificity for said target when compared with an antibody which, for example, specifically recognises both the target and a homologous protein.

A first aspect of the present invention provides a method for the differential diagnosis of small cell and non-small cell lung cancer, comprising determining the concentration of three or more biomarkers selected from the list consisting of CRP, D-dimer, NGAL, NSE and VEGF in a sample obtained from the subject, and establishing the significance of these levels.. Preferred combinations of the current invention include, but are not limited to, CRP, D-dimer and NGAL; CRP, D-dimer, NGAL and NSE; CRP, D-dimer, NGAL and VEGF; or CRP, D-dimer, NSE, NGAL and VEGF.

The term "CRP" as used herein refers to C reactive protein (UniProt P02741). The term "D-dimer" as used herein refers to the fibrin degradation product. The term "NGAL" as used herein refers Neutrophil gelatinase-associated lipocalin (UniProt P80188), also known as lipocalin 2 (LCN2). The term "NSE" as used herein refers to neuron specific enolase (UniProt P09104), also known as gamma enolase and enolase 2 (ENO2). The term "VEGF" as used herein refers to Vascular Endothelial Growth Factor A (UniProt P15692), also known as vascular permeability factor (VPF).

In a further embodiment of the current invention the concentration of one or more additional biomarkers selected from the list comprising soluble Tumour Necrosis Factor Receptor 1 (sTNFR1), Epidermal Growth Factor (EGF), Monocyte Chemotactic Protein 1 (MCP-1), Interleukin 6 (IL-6) and Interleukin 8 (IL-8) is also determined.

The term "sTNFR1" as used herein refers to soluble Tumour Necrosis factor Receptor 1 (UniProt P19438). The term "EGF" as used herein refers to Epidermal Growth Factor (UniProt 01133). The term "MCP-1" as used herein refers to Monocyte Chemotactic Protein 1 (Uniprot P13500). The term "IL-6" as used herein refers to interleukin 6 (UniProt P05231). The term "IL-8" as used herein refers to interleukin 8 (UniProt P10145).

The determination of the level of biomarkers in the sample may be determined by immunological methods such as an immunoturbidimetric assay or ELISA based assay. Preferably, the methods of the present invention use a solid state device for determining the level of biomarkers in the sample isolated from the patient. The solid state device comprises a substrate having an antibody that binds specifically to a biomarker immobilised upon it. Such antibodies may be immobilised at discreet areas of an activated surface of the substrate. The solid state device may perform multi-analyte assays such that the level of one biomarker in a sample isolated from the patient may be determined simultaneously with the level of one or more further biomarkers of interest in the sample. In this embodiment, the solid state device has a multiplicity of discrete reaction sites each bearing a desired antibody covalently bound to the substrate, and in which the surface of the substrate between the reaction sites is inert with respect to the target biomarker. The solid state, multi-analyte device may therefore exhibit little or no non-specific binding. Wherein one or more of the biomarkers is not compatible with a multi-analyte format they can be determined simultaneously, or indeed separately, using a suitable format such as ELISA or immunoturbidimetric assay.

A device that may be used in the invention may be prepared by activating the surface of a suitable substrate, and applying an array of antibodies on to discrete sites on the surface. If desired, the other active areas may be blocked. The ligands may be bound to the substrate via a linker. In particular, it is preferred that the activated surface is reacted successively with an organosilane, a bifunctional linker and the antibody. The solid state device used in the methods of the present invention may be manufactured according to the method disclosed in, for example, GB patent number GB2324866. Preferably, the solid state device used in the methods of the present invention is the Biochip Array Technology system (BAT) (available from Randox Laboratories Limited, Crumlin, Northern Ireland). More preferably, the Evidence Evolution, Evidence Investigator and Multistat apparatus (also available from Randox Laboratories) may be used to determine the levels of biomarkers in the sample.

A biomarker present in a sample isolated from a patient having cancer may have levels which are different to that of a control. However, the levels of some biomarkers that are different compared to a control may not show a strong enough correlation with cancer such that they may be used to diagnose cancer with an acceptable accuracy. If two or more biomarkers are to be used in the diagnostic method a suitable mathematical or machine learning classification model, such as logistic regression equation, can be derived. The significance of the levels of the biomarkers can be established by inputting into said model. Such a classification model may be chosen from at least one of decision trees, artificial neural networks, logistic regression, random forests, support vector machine or indeed any other method developing classification models known in the art.

Accuracy of a diagnostic method is best described by its receiver-operating characteristics (ROC) [15]. The ROC graph is a plot of all of the sensitivity/specificity pairs resulting from continuously varying the decision threshold over the entire range of data observed.

A ROC plot depicts the overlap between the two distributions by plotting the sensitivity versus 1 - specificity for the complete range of decision thresholds. On the y-axis is sensitivity, or the true-positive fraction defined as [(number of true-positive test results)/ (number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. Qualitatively, the closer the plot is to the upper left corner, the higher the overall accuracy of the test.

One convenient goal to quantify the diagnostic accuracy of a laboratory test is to express its performance by a single number. The most common global measure is the area under the curve (AUC) of the ROC plot. The area under the ROC curve is a measure of the probability that the perceived measurement will allow correct identification of a condition. By convention, this area is typically ≥ 0.5. Values range between 1.0 (perfect separation of the test values of the two groups) and 0.5 (no apparent distributional difference between the two groups of test values). Any result < 0.5 is considered to be worse than a random guess. The area does not depend only on a particular portion of the plot such as the point closest to the diagonal or the sensitivity at 90% specificity, but on the entire plot. This is a quantitative, descriptive expression of how close the ROC plot is to the perfect one (area = 1.0). In the context of the present invention, the two different conditions are whether a patient has SCLC or NSCLC. Table 3 shows the areas under the curve for single markers and two or more marker combinations. In a clinical setting, since SCLC is approximately 20% of the number of total cases of lung cancer and NSCLC is more prevalent, it would be desirable to assign SCLC with 100% sensitivity. This means that the majority of subjects, who will end up outside of this category, can have SCLC definitely ruled out and start appropriate treatment for NSCLC.

In preferred embodiments of the current invention the significance of the levels of biomarkers are established by inputting the values into a suitable classification model. Most preferably the classification models used are decision trees or logistic regression. A decision tree may be grown where a decision branch is grown from each node (subpopulation) to divide the population into classification groups. Example 1 and Figures 3 and 4 represent examples of trees that were grown using the data described in this invention, which could correctly classify Lung Cancer patients with a relatively small error. Using suitable cut-off values the data may be interpreted by an individual or more preferably the biomarker data may be inputted manually or automatically into dedicated software which, by means of a suitable classification model or algorithm, produces an output indicating whether the sample should be classified in the SCLC or NSCLC groups.

In a further aspect the biomarker combinations of the current invention allow the monitoring of the development of lung cancer within an individual through serial testing of samples from said individual over an extended period. For example, routine determination of the levels of biomarkers of the preferred combinations could detect the changes from healthy control values or previous levels from the same patient, which are indicative of the development of lung carcinoma. A further change in levels could then be indicative of the progression of the disease to a later stage.

A further aspect of the present invention is a method of determining the efficacy of a treatment for lung cancer comprising determining the levels of three or more biomarkers selected from the list consisting of CRP, D-dimer, NGAL, NSE and VEGF in a sample from a treated patient, and comparing levels with those from a healthy control or with levels from the same patient before treatment, wherein dependent on the biomarker, either an increase or decrease in level indicates the effectiveness of the treatment. The treatment can be for example, a drug treatment, a radiotherapy based treatment or a surgical intervention. In a preferred method for determining the efficacy of a treatment for lung cancer the biomarkers consist of CRP, D-dimer and NGAL. Wherein the treatment is a drug treatment, the method of determining the efficacy of the drug treatment for lung cancer would comprise determining the levels of biomarkers, for example CRP, D-dimer and NGAL in a sample from a patient treated with the drug, and comparing biomarker levels with those from a healthy control or with levels from the same patient before treatment with the drug, wherein, dependent on the biomarker, either an increase or decrease in level indicates the effectiveness of the drug treatment. Combinations of the current invention may also be used to predict the efficacy in advance of a treatment based on the significance of the measured levels.

### METHODS

Lung Cancer patient serum samples for the discovery set were provided by ProMedDx, Norton, MA, United States. Patient samples for the verification set were provided by Analytes Ltd., Torokbalint, Hungary. Samples were collected in accordance with local ethical and governance requirements. All samples were supplied pre-anonymised and with relevant clinical notes, including any cytological diagnosis and subtyping information. In total the discovery set contained 11 SCLC and 30 NSCLC samples while the verification set contained 5 SCLC and 11 NSCLC samples. Samples were aliquoted and stored at -80 C prior to analysis on biochip.

The Cerebral Array II was used as per manufacturer's instructions (Product code EV3637, Randox Laboratories Ltd., Crumlin, Northern Ireland) to determine levels of CRP, NSE, D-dimer, NGAL and VEGF in patient serum samples. Briefly, Calibrators/controls/samples were pre-diluted 1:8 (35 µl plus 245 µl) with dilution buffer in a clean vessel. 100 µl of assay diluent was added to each well followed by 200 µl of the pre-diluted calibrator/control/sample as required. The carrier was then incubated at 37°C for 45 minutes at 370 rpm on a thermoshaker. Following the incubation the liquid was decanted and each well washed. Two quick washes and four two-minute soaks were performed. The liquid was then decanted and the carrier tapped onto lint free tissue paper. 300 µl of conjugate was then added to each reaction well and the carrier incubated at 37°C for a further 45 minutes at 370 rpm on the thermoshaker. Following the incubation the liquid was again decanted and each well washed. Two quick washes and four two-minute soaks were performed. Luminol-EV805 and Peroxide were mixed to form the signal reagent (1:1). 250 µl of signal reagent-EV805 was added to each reaction well. This was incubated for 2 minutes and protected from light. Each carrier was then imaged on the Evidence Investigator system (Randox Laboratories Ltd., Crumlin, Northern Ireland) and the results are processed by the dedicated software onboard.

Levels of sTNFR1, EGF, MCP-1, IL-8 and IL-6 were determined in patient serum samples using the Cytokine and growth factors array as per manufacturer's instructions (Product code EV3513, Randox Laboratories Ltd., Crumlin, Northern Ireland). Briefly, Calibrators/controls/samples were pre-diluted 1:8 (35 µl plus 245 µl) with dilution buffer in a clean vessel. 100 µl of assay diluent was added to each well followed by 200 µl of the pre-diluted calibrator/control/sample as required. The carrier was then incubated at 37°C for 60 minutes at 370 rpm on a thermoshaker. Following the incubation the liquid was decanted and each well washed. Two quick washes and four two-minute soaks were performed. The liquid was then decanted and the carrier tapped onto lint free tissue paper. 300 µl of conjugate was then added to each reaction well and the carrier incubated at 37°C for a further 60 minutes at 370 rpm on the thermoshaker. Following the incubation the liquid was again decanted and each well washed. Two quick washes and four two-minute soaks were performed. Luminol-EV805 and Peroxide were mixed to form the signal reagent (1:1). 250 µl of signal reagent-EV805 was added to each reaction well. This was incubated for 2 minutes and protected from light. Each carrier was then imaged on the Evidence Investigator system (Randox Laboratories Ltd., Crumlin, Northern Ireland) and the results are processed by the dedicated software onboard.

The invention is further described with reference to the following non-limiting examples:

### Example 1

An algorithm can be applied in the form of a decision tree as follows (see also Figures 3 and 4):
Decision 1: Subjects are classified according to a cut-off value for C-related peptide (CRP) levels; those of <12 mg/L are assigned to the NSCLC group and those with CRP ≥12 mg/L are assigned for further categorisation by Decision 2.
Decision 2: Subjects with D-dimer <123 ng/ml are assigned for further categorisation by Decision 3A (NGAL). Subjects with D-dimer ≥123 ng/ml are assigned for further categorisation by Decision 3B (NSE).
Decision 3A: Subjects with NGAL <1400 ng/ml are assigned to the SCLC group. Subjects with NGAL ≥1400 ng/ml are assigned for further categorisation by Decision 4A.
Decision 3B: Subjects with NSE <3 ng/ml are assigned to the NSCLC group. Subjects with NSE ≥3 ng/ml are assigned for further categorisation by Decision 4B.
Decision 4A: Subjects with VEGF ≥100 pg/ml are assigned to the NSCLC group. Subjects with VEGF<100 pg/ml are assigned to the SCLC group.
Decision 4B: Subjects with VEGF <100 pg/ml are assigned to the NSCLC group. Subjects with VEGF≥100 pg/ml are assigned to the SCLC group.

The present invention has identified that if the above algorithm is applied to values for the serum concentration for the analytes shown, then cases of SCLC from a population of subjects with lung cancer can be determined with 100% sensitivity and 96.7% specificity, giving PPV of 91.7% and NPV of 100% (Figure 3 and Table 1). This reflects that out of 41 lung cancer subjects in the initial discovery set, only one case (of NSCLC as SCLC) was incorrectly classified by the algorithm. In a small independent verification set (Figure 4 and table 2), the algorithm achieved 100% sensitivity and 81.8% specificity, giving PPV of 71.4% and NPV of 100%.

**Table 1 - Results calculated when a decision tree based on cut-off values for CRP, D-dimer, NGAL, NSE and VEGF is applied to the discovery set of patient data (figure 3).**

| | **% Sensitivity** | **% Specificity** | **% Positive Predictive Value** | **% Negative Predictive Value** | **Decision** |
|---|---|---|---|---|---|
| **decision** | **100** | **96.7** | **91.7** | **100** | **Yes: 11 SCLC/1 non- SCLC** |
| | | | | | **No: 0 SCLC/29 non-SCLC** |
| **-VEGF decision** | **90.9** | **93.3** | **83.3** | **96.6** | **Yes: 10 SCLC/2 non- SCLC** |
| | | | | | **No: 1 SCLC/28 non-SCLC** |

**Table 2 - Results calculated when a decision tree based on cut-off values for CRP, D-dimer, NGAL and NSE is applied to the verification set of patient data (figure 4).**

| **% Sensitivity** | **% Specificity** | **% Positive Predictive Value** | **% Negative Predictive Value** | **Decision** |
|---|---|---|---|---|
| **100** | **81.8** | **71.4** | **100** | **Yes: 5 SCLC/2 non-SCLC** |
| | | | | **No: 0 SCLC/9 non-SCLC** |

### Example 2

Regression analysis was carried out on the initial discovery set using a SPSS software package (IBM Corporation, Armonk, New York, United States) for each of the individual biomarkers CRP, D-dimer, NSE, NGAL and VEGF (Table 3). A further linear regression analysis was carried out using the "Enter" method to input the biomarkers CRP, D-dimer, NSE, NGAL and VEGF into a regression model to show how they work together to distinguish between SCLC and NSCLC (Table 3). The combination of all 5 biomarkers together achieved an AUC value of 0.870 while the highest AUC (0.924) was the result of a combination consisting of CRP, D-dimer and NSE.

**Table 3 - AUC values calculated for altered levels of individual and combinations of biomarkers in differentiating SCLC from NSCLC.**

| Test result variable (s) | Area under the Curve | Sensitivity (%) | Specificity (%) |
|---|---|---|---|
| CRP/D-dimer/NSE | 0.924 | 100 | 80 |
| D-dimer/NSE | 0.885 | 100 | 57 |
| D-dimer/NGAL/NSE | 0.876 | 100 | 53 |
| CRP/D-dimer/NGAL/NSE | 0.870 | 91 | 80 |
| CRP/D-dimer/NSE/NGAL/VEGF | 0.870 | 91 | 77 |
| CRP/D-dimer | 0.858 | 91 | 80 |
| CRP/D-dimer/NSE/VEGF | 0.852 | 100 | 60 |
| CRP/NSE | 0.838 | 100 | 47 |
| CRP/NGAL/NSE | 0.836 | 100 | 40 |
| CRP/D-dimer/NGAL | 0.830 | 91 | 77 |
| CRP/NGAL/NSE/VEGF | 0.830 | 91 | 50 |
| CRP/D-dimer/NGAL/VEGF | 0.827 | 91 | 64 |
| CRP/NSE/VEGF | 0.818 | 100 | 50 |
| VEGF/CRP/D-dimer | 0.815 | 100 | 53 |
| VEGF/NGAL/CRP | 0.809 | 91 | 50 |
| D-dimer/NSE/VEGF | 0.806 | 100 | 40 |
| D-dimer/NGAL/NSE/VEGF | 0.794 | 100 | 40 |
| CRP/VEGF | 0.791 | 100 | 47 |
| CRP/NGAL | 0.782 | 91 | 67 |
| NSE | 0.738 | 82 | 53 |
| NGAL/NSE | 0.736 | 82 | 57 |
| NGAL/NSE/VEGF | 0.721 | 82 | 50 |
| VEGF/D-dimer/NGAL | 0.709 | 82 | 57 |
| D-dimer/VEGF | 0.703 | 100 | 37 |
| CRP | 0.700 | 100 | 40 |
| NSE/VEGF | 0.700 | 100 | 23 |
| VEGF | 0.648 | 82 | 30 |
| NGAL | 0.461 | 73 | 33 |
| D-dimer | 0.315 | 82 | 17 |

An additional regression analysis was carried out using the "Enter" method to input the biomarkers above, CRP, D-dimer, NSE, NGAL and VEGF, and also six additional biomarkers (SCC, sTNFR1, EGF, MCP-1, IL-6 and IL-8). An AUC value of 0.903 was calculated for this 11 biomarker combination.

## Claims

1. A method for aiding in the differential diagnosis of small cell lung cancer (SCLC) and non-small cell lung cancer (NSCLC) in a subject with lung cancer, comprising,
a) Determining the concentration of CRP, D-dimer and NGAL in an *ex vivo* serum or plasma sample obtained from the subject, and:
b) Establishing the significance of the measured levels of biomarkers by
inputting the obtained values into a suitable classification model or algorithm which produces an output indicating whether the sample should be classified as SCLC or NSCLC.

2. The method of claim 1 wherein one or more additional biomarker is determined, said biomarker being chosen from NSE and VEGF.

3. The method of Claims 1-2 wherein the concentration of one of more additional biomarker selected from the list consisting of soluble Tumour Necrosis Factor Receptor 1, Epidermal Growth Factor, Monocyte Chemotactic Protein 1, Interleukin 6 and Interleukin 8 is also determined.

4. The method according to Claims 1-3, wherein the significance of the measured levels of biomarkers is established by inputting the measured values into a classification model chosen from at least one of decision trees, artificial neural networks, logistic regression, random forests, support vector machines or any other method developing classification models known in the art.

5. The use of a solid-state device comprising at least three probes, each probe independently specific for a biomarker selected from CRP, D-dimer and NGAL, to aid in the differential diagnosis of small cell and non-small cell lung cancer, wherein the sample type to be analysed is an *ex vivo* serum or plasma sample.

6. A method of determining the efficacy of a treatment for lung cancer comprising, measuring the amount of CRP, D-dimer and NGAL in an ex vivo sample taken from a treated patient, and comparing the levels with levels from a healthy control or with levels from the same patient before treatment, wherein a change in levels indicates the efficacy of the treatment.

## Patentansprüche

1. Verfahren zur Unterstützung der Differentialdiagnose von kleinzelligem Lungenkrebs (SCLC) und nicht-kleinzelligem Lungenkrebs (NSCLC) bei einem Patienten mit Lungenkrebs, umfassend:
a) Bestimmung der Konzentration von CRP, D-Dimer und NGAL in ex vivo vom Probanden erhaltene Serum- oder Plasmaprobe und:
b) Ermittlung der Bedeutung der gemessenen Biomarkerwerte durch Eingabe der erhaltenen Werte in ein geeignetes Klassifizierungsmodell oder einen geeigneten Algorithmus, der eine Ausgabe erzeugt, die angibt, ob die Probe als SCLC oder NSCLC klassifiziert werden soll.

2. Verfahren nach Anspruch 1, wobei ein oder mehrere zusätzliche Biomarker bestimmt werden, wobei der Biomarker aus NSE und VEGF ausgewählt wird.

3. Verfahren nach Anspruch 1-2, wobei die Konzentration eines oder mehrerer zusätzlicher Biomarker, ausgewählt aus der Liste bestehend aus löslichem Tumornekrosefaktorrezeptor 1, epidermalem Wachstumsfaktor, chemotaktischem Monozytenprotein 1, Interleukin 6 und Interleukin 8, ebenfalls bestimmt wird.

4. Verfahren nach den Ansprüchen 1 bis 3, wobei die Signifikanz der gemessenen Mengen an Biomarkern durch Eingabe der gemessenen Werte in ein Klassifizierungsmodell ermittelt wird, das aus mindestens einem von Entscheidungsbäumen, künstlichen neuronalen Netzen, logistischer Regression, zufälligen Wäldern, Unterstützung von Vektormaschinen oder einer anderen Methode zur Entwicklung von Klassifizierungsmodellen, die auf dem Fachgebiet bekannt sind.

5. Verwendung einer Festkörpervorrichtung mit mindestens drei Sonden, wobei jede Sonde unabhängig für einen aus CRP, D-Dimer und NGAL ausgewählten Biomarker spezifisch ist, um die Differentialdiagnose von kleinzelligem und nicht kleinzelligem Lungenkrebs zu unterstützen; wobei der zu analysierende Probentyp eine Ex-vivo-Serum- oder Plasmaprobe ist.

6. Verfahren zur Bestimmung der Wirksamkeit einer Behandlung gegen Lungenkrebs, umfassend das Messen der Menge an CRP, D-Dimer und NGAL in einer Ex-vivo-Probe, die einem behandelten Patienten entnommen wurde, und das Vergleichen der Spiegel mit Spiegeln einer gesunden Kontrolle oder mit Spiegel von demselben Patienten vor der Behandlung, wobei eine Änderung der Spiegel die Wirksamkeit der Behandlung anzeigt.

## Revendications

1. Procédé pour aider au diagnostic différentiel du cancer du poumon à petites cellules (SCLC) et du cancer du poumon non à petites cellules (NSCLC) chez un sujet atteint d'un cancer du poumon, comprenant:
a) Détermination de la concentration de CRP, D-dimère et NGAL dans un ex vivo échantillon de sérum ou de plasma obtenu du sujet, et:
b) Établir l'importance des niveaux mesurés de biomarqueurs par entrer les valeurs obtenues dans un modèle ou algorithme de classification approprié qui produit une sortie indiquant si l'échantillon doit être classé comme SCLC ou NSCLC.

2. Procédé selon la revendication 1, dans lequel un ou plusieurs biomarqueurs supplémentaires sont déterminés, ledit biomarqueur étant choisi parmi NSE et VEGF.

3. Procédé selon les revendications 1 à 2, dans lequel la concentration d'un ou de plusieurs biomarqueurs supplémentaires sélectionnés dans la liste consistant en récepteur de facteur de nécrose tumorale soluble 1, facteur de croissance épidermique, protéine chimiotactique monocytaire 1, interleukine 6 et interleukine 8 est également déterminée.

4. Procédé selon les revendications 1 à 3, dans lequel l'importance des niveaux mesurés de biomarqueurs est établie en introduisant les valeurs mesurées dans un modèle de classification choisi parmi au moins l'un des arbres de décision, les réseaux de neurones artificiels, la régression logistique, les forêts aléatoires, prendre en charge des machines à vecteurs ou toute autre méthode développant des modèles de classification connus dans l'art.

5. L'utilisation d'un dispositif à l'état solide comprenant au moins trois sondes, chaque sonde étant indépendamment spécifique d'un biomarqueur choisi parmi le CRP, le D-dimère et le NGAL, pour faciliter le diagnostic différentiel du cancer du poumon à petites cellules et non à petites cellules, dans lequel le type d'échantillon à analyser est un échantillon de sérum ou de plasma ex vivo.

6. Procédé de détermination de l'efficacité d'un traitement du cancer du poumon comprenant, la mesure de la quantité de CRP, de D-dimère et de NGAL dans un échantillon ex vivo prélevé sur un patient traité, et la comparaison des niveaux avec les niveaux d'un contrôle sain ou avec niveaux du même patient avant le traitement, où un changement de niveau indique l'efficacité du traitement.
